# EUROPEAN PATENT SPECIFICATION

(11) **EP 1 374 813 B1**
(45) Date of publication and mention of the grant of the patent: **08.10.2008**
(21) Application number: 03254086.6
(22) Date of filing: 27.06.2003
(51) Int. Cl.: A61F 13/02

(54) **Adhesive bandage having a selectively placed layer**
Klebende Bandage mit einer selektiv plazierten Schicht
Pansement adhesif avec une couche placée séléctivement

(30) Priority: 28.06.2002 US 184490
(43) Date of publication of application: 02.01.2004
(73) Proprietor: JOHNSON & JOHNSON CONSUMER COMPANIES, INC., Skillman, NJ 08558 (US)
(72) Inventor: Siegwart, Kathleen Ann, Milford, New Jersey 08848 (US); Pedro, Gabriel Jr., Belle Mead, New Jersey 08502 (US); Jackson, Peter W., Hampton, New Jersey 08827 (US)
(74) Representative: Mercer, Christopher Paul

(56) References cited:
- EP-A- 0 099 748
- EP-A- 0 673 657
- WO-A-90/03155
- US-A- 5 244 457
- US-A- 5 512 041
- US-A- 5 695 777

## Description

### BACKGROUND OF THE INVENTION

### Field of the Invention

The present invention relates to adhesive bandages for application to skin wounds, irritations, abrasions, bruises and the like. More specifically, this invention relates to adhesive bandages that include a layer selectively placed to provide a desired benefit such as water impermeability or improved cushioning and protection of the wound to which the bandage is applied in the use thereof.

### Description of the Prior Art

For many years, adhesive bandages have been sold for the purpose of assisting in the treatment and/or protection of cuts, bruises, abrasions and similar injuries to the skin. These bandages cover and protect the wound while it heals. Such bandages comprise a backing material, one major surface of which is coated with an adhesive. A wound-contacting pad, which in use overlays the wound, is secured to the adhesive coated surface of the backing (usually, but not always, in the generally central region thereof) by a portion of the adhesive composition. The remaining portions of the adhesive composition serve, during use, to adhere the bandage to the skin surrounding the wound site. In some instances, disposable adhesive bandages are used to deliver active materials, e.g., antibiotics, anesthetics, soothing agents and the like, to the site of the injury.

Backing materials made from polymers such as polyethylene and polyvinylchloride are known in the art. Such backing materials have the desirable property of being liquid water impermeable. On the other hand, these backing materials are also impermeable to gases such as oxygen and water vapor. If water vapor can not evaporate from the skin under a backing material, the skin tends to macerate and become uncomfortable. Therefore, backing materials which are both liquid water impermeable and water vapor impermeable are typically perforated to allow water vapor to evaporate from the skin, i.e., to allow the skin to "breathe". Such perforated backing materials may, however, permit environmental elements such as water or dirt to reach a wound. This, in turn, may delay the healing process.

The development of wound bandage technology has been aided by recent advances in biocompatible synthetic materials, including thin films and adhesives that are impermeable to liquids, such as water, and permeable to gases, such as oxygen and water vapor. It has been shown that providing a sterile wound covering that is permeable to water vapor and impermeable to liquid water is an aid to healing of the wound. Various polyurethane films are available for use as backing materials in bandages which films are impermeable to water and similar liquids but are permeable to water vapor and other gases such as oxygen. Polyurethane films having a thickness of less than about 2 mils allow for free diffusion of oxygen, water vapor and other gases through their molecular matrices while at the same time providing impermeability to liquids such as water.

Currently, there are products on the market that comprise semi-permeable backing materials that are water vapor permeable but impermeable to liquid water and dirt. Examples of such prior art products include BAND-AID® Brand Water Block Plus* bandages, and NEXCARE® waterproof bandages. These bandages are known in the art and sold as water impermeable bandages.

The aforementioned backing materials are known in the art as "semi-permeable" backing materials for the reason that they are permeable to water vapor and oxygen (thus allowing the skin and wounded area covered by the bandage to "breathe") and impermeable to liquid water (thus substantially reducing the risk of contaminating the wound or delaying the healing process as a result of the incursion of water and the like liquids through the thickness of the backing material). The above-described semi-permeable backing materials are generally expensive. In addition, the desired moisture vapor transmission rates are generally achieved by reducing the thickness of the backing material. This reduction in thickness, however, is known to make the bandage more difficult to handle, especially when applying it to a wound or other injury.

Those skilled in the art will recognize that the entirety of the backing material used in the above-mentioned prior art bandages is semi-permeable. Thus, for example, in a typical bandage having a width of 2.54 cm (one (1) inch) and a length of 7.62 cm (three (3) inches), the entire 7.62 square cm (three square inch) strip of backing material used to form the bandage is semi-permeable.

United States Patent Number 5,633,070 discloses a backing material which is useful in the manufacture of adhesive bandages. This backing material is formed by laminating a semi-permeable film, i.e., a film having water vapor permeability and liquid water impermeability, to a flexible nonwoven fabric also having water vapor permeability and liquid water impermeability. Bandages using the backing material disclosed by the '070 patent allow skin respiration and prevent permeation of water through the thickness of the bandage to the wound site. The water vapor permeable/liquid water impermeable film and the water vapor permeable/liquid water impermeable fabric are coextensive in length and width. These backing materials are expensive and their manufacture involves a laminating step.

D1 discloses a moisture vapour transmitting wound dressing that comprises a backing layer, which has an adhesive on one of its surfaces and in which at least one of the backing layer and adhesive layer is continuous so as to provide a barrier both to bacteria and to liquid water when the dressing is in place on a wound. The wound dressing further comprises an absorbent pad positioned on the adhesive side of the backing layer. The area of the absorbent pad is less than that of the backing layer so that an adhesive margin remains exposed on two, or four, sides of the pad such that the backing layer may be adhered to the skin surrounding the wound area.

D2 discloses a vented dressing comprising a thin vapor-permeable, liquid-impermeable sheet material having a wound-covering portion for placement directly over the wound and a portion which is intended to be secured to the whole skin surrounding the wound. The sheet material has a coating of a pressure-sensitive adhesive extending substantially entirely over one surface for securing the dressing to the body.

D3 discloses a wound dressing comprising: an outer vapor permeable layer permitting transpiration of fluid from the dressing; an intermediate layer of hydrogel adapted for absorbing wound exudate; a wound-contacting layer for separating the intermediate hydrogel layer from the wound; wicking means associated with the wound-contacting layer for conducting exudate from the wound to the hydrogel; and a therapeutic agent retained in the dressing by the wound-contacting layer.

D4 discloses a wound dressing comprising: a backing sheet coated with a pressure-sensitive adhesive; an absorbent pad adhered to the adhesive; and a net extending across the pad and adhered to the adhesive. The perimeter of the pad is completely surrounded by a portion of the adhesive. The backing sheet comprises a strip of stretchable, liquid-permeable, non-woven fabric and a semi-occlusive film. The fabric strip is preferably disposed on an interior side of the film and carries the adhesive. The film defines an external face of the dressing and covers the entire surface area of the fabric strip.

There is a need for a semi-permeable bandage that protects the wound from liquid water, allows the passage of gases, e.g., oxygen and water vapor, to and from the wound, is less expensive to manufacture, easier to apply, flexible, and comfortable to wear. There is also a need for an adhesive bandage with improved wound and bruise cushioning properties.

The importance of a wound-contacting pad in an adhesive bandage is well established. The wound-contacting pad absorbs blood and other body exudate from the wound site. It also provides coverage of the wound and helps protect it from dirt, microorganisms, and re-injury. The wound-contacting pad often includes medicaments, such as disinfectants, antimicrobial agents, antibiotics and the like, An example of a wound-contacting pad which can deliver medicaments or other desirable active ingredients to a wound site is disclosed in U.S. Patent No. 5,814,031.

As used herein, the term "selective placement of a layer of material" means that a layer of material is secured to a backing material and said layer of material is not coextensive, in at least one direction (usually the length direction), with the backing material.

As used herein, a "semi-permeable material" is a material which is permeable to water vapor and other gases, such as oxygen, and is impermeable to water and other liquids.

As used herein, a "breathable material" is a material which permits the passage therethrough of water vapor and other gases such as oxygen. It will be understood that certain materials, such as woven fabrics, nonwoven fabrics and fibrous layers (for example, the fibrous layers of the kind used in wound-contacting pads) are inherently breathable. It will be further understood that materials which are inherently non-breathable may be treated to make them breathable. Polyolefin, polyvinylchloride, ethylene-vinyl acetate and the like polymeric films may be treated, e.g., by perforating, to make them breathable.

### SUMMARY OF THE INVENTION

Adhesive bandages in accordance with the teachings of the present invention comprise a backing material, an adhesive, a selectively placed layer of material further comprising a semi-permeable film and a wound-contacting pad.

In accordance with a first embodiment of the present invention, an adhesive bandage comprises a backing material, an adhesive, a selectively placed layer comprising a semi-permeable film, and a wound-contacting pad; each of said backing material, said semi-permeable film and said wound contacting pad have a respective length and width; said backing material has a first and second major surface; said selectively placed layer is secured to said backing material by said adhesive; at least the length of said selectively placed layer is less than the length of said backing to which it is secured; and said wound-contacting pad is secured to said selectively placed layer.

In a second embodiment, an adhesive bandage according to the present invention is provided wherein the length of the selectively placed layer is less than the length of the backing material and the width of the semi-permeable film is substantially the same as the width of the backing material.

In a third embodiment, an adhesive bandage according to the present invention is provided wherein the length of the selectively placed semi-permeable film is less than the length of the backing material and the width of the semi-permeable film is less than the width of the backing material.

In a fourth embodiment, an adhesive bandage according to the present invention is provided wherein the length and width of the selectively placed layer are less than the length and width of the backing material and the wound-contacting pad has a length, which is less than the length of the selectively placed layer.

In a fifth embodiment, an adhesive bandage according to the present invention is provided wherein the length and width of the selectively placed layer are less than the length and width of the backing layer and the length and width of the wound-contacting pad are less than the length and width of the selectively placed layer.

In accordance with an alternative embodiment of the present invention, the selectively placed layer further comprises cushioning means for cushioning a wound. The selectively placed layer of wound-cushioning material may comprise, for example, a polymeric foam, a woven fabric, a non-woven fabric, or a polymeric film which has been treated, as by embossing, to have a thickness sufficient to provide wound-cushioning properties. The cushioning material may include a bi-layer polymeric film which defines a pocket or, preferably, a plurality of small pockets, in which a gas is trapped. The wound-cushioning material has at least one dimension, e.g., its length, which is less than the corresponding dimension, i.e., the length, of the backing material. Depending on the desired properties of the bandage, the wound-cushioning material may be semi-permeable but this is not required.

As used herein, the term "semi-permeable film" also includes semi-permeable foams. The adhesive, is preferably a skin-compatible, medically acceptable pressure-sensitive adhesive, and is applied to and carried by one major surface of the backing material. As outlined above, the selectively placed layer is secured to the backing and the wound-contacting pad is secured to the selectively placed layer. As is known, the remaining portions of adhesive serve to affix the bandage to the skin during use. In use, the adhesive bandage of the present invention protects wounds from contamination because it is substantially impermeable to liquid water, microorganisms, and particles of dirt. The bandage also provides a good healing environment for wounds. In use, the adhesive bandage of the alternative embodiment of the invention provides cushioning over and beyond the cushioning attributable to the presence of the usual wound-contacting pad. If the wound-cushioning layer is semi-permeable, then the possibility of environmental elements such as water and dirt reaching the wound site will be substantially reduced. Bandages in accordance with this alternative embodiment of the present invention also provide a good healing environment for wounds.

### DETAILED DESCRIPTION OF THE DRAWINGS

The invention will be more clearly understood by reference to the accompanying drawings on which:
FIG. 1 is an exploded perspective, with some portions upturned and other portions cut away, of one embodiment of an adhesive bandage in accordance with the present invention;
FIG. 2 is a perspective view of the bandage of FIG. 1;
FIG. 3 is a longitudinal cross-section taken along line 3-3 of FIG. 2;
FIG. 4 is an exploded perspective, with some portions upturned and other portions cut away, of an embodiment of an adhesive bandage in accordance with the present invention;
FIG. 5 is a perspective view of the bandage of FIG. 4;
FIG. 6 is a longitudinal cross-section taken along line 6-6 of FIG. 5;
FIG. 7 is a perspective view, with certain portions upturned and certain portions cut away, of an embodiment of an adhesive bandage according to the present invention;
FIG. 8 is a perspective view of an adhesive bandage wherein the selectively placed layer comprises a wound-cushioning material; and
FIG. 9 is a longitudinal cross-section taken along line 9-9 of FIG. 8.

### DETAILED DESCRIPTOR OF PREFERRED EMBODIMENTS

Backing materials useful in the practice of the present invention include, but are not limited to, polymeric films, including polyolefin films such as polyethylene and polypropylene films; polyvinylchloride films; and ethylene-vinyl acetate films. Other useful backing materials include nonwoven fabrics, woven fabrics, and laminates of polymeric films with woven fabrics or nonwoven fabrics. A woven backing material particularly useful for practice of the invention has polyester yarns such as polyethylene terephthalate or polybutylene terephthalate yarns in the warp direction and polyamide yarns, such as nylon 6 or nylon 6,6 yarns, in the fill direction. Alternatively, the woven backing material may have polyethylene terephthalate yarns in the warp direction and polybutylene terephthalate yarns in the fill direction. Such woven backings are known and are commercially available. If breathability is desired in a backing material, and said backing material is not inherently breathable, then the desired breathability may be obtained by perforating the backing material as is known in the art, Backing materials for use in the practice of the present invention are preferably breathable; however, non-breathable backing materials may be used, if desired.

Apertured films are useful as backing materials in the practice of the invention. Such apertured films are breathable films. Particularly useful apertured films include Vispore® Brand apertured film supplied by Tredegar under the designations Tredegar X-6799, Tredegar X-6845, Tredegar X-6923, Tredegar X-6944, and Tredegar X-6844. Apertured films may be made from any polymeric material including, but not limited to polyethylene, metallocene catalyzed polyethylene, polypropylene, polyolefin copolymers, and ethylene vinyl acetate copolymers.

In order to be more cost effective than current commercially available adhesive bandages whose entire backing material comprises a semi-permeable film, the adhesive bandages of the present invention comprise a selectively placed layer which is smaller in at least one direction, typically the longitudinal direction, than the backing material to which it is secured. In other words, at least some portion of the backing material has no selectively placed layer secured thereto. In certain embodiments, both the length and width of the selectively placed layer are less than the length and width, respectively, of the backing material. It will be understood that the amount of backing material covered by the selectively placed layer may vary. Generally, the selectively placed layer covers a maximum of about 86% of the backing material, Preferably, the selectively placed layer covers a maximum of about 70% of the backing material. More preferably, the selectively placed layer covers a maximum of about 53% of the backing material. Even more preferably, the selectively placed layer covers a maximum of about 46% of the backing material.

In one embodiment of the present invention, the selectively placed layer is a semi-permeable film. This selectively placed semi-permeable film prevents liquid water from reaching the wound, yet allows water to evaporate from the wound. Examples of suitable semi-permeable layers include, but are not limited to, semi-permeable polyurethane films, semi-permeable polyurethane foams, semi-permeable polyolefin films, semi-permeable polyetheramide films available under the PEBAX trademark and semi-permeable copolyester films available under the HYTREL trademark. Semi-permeable polyurethane films are preferred.

As is known in the art, the wound-contacting pad of an adhesive bandage protects the wound from contamination by dirt. The absorbent pad may be made from various materials including rayon fibers; natural fibers, such as, but not limited to, cotton and wood pulp fibers, and synthetic fibers, such as, but not limited to, polyester, polyamide, and polyolefin fibers. Synthetic fibers comprising two or more polymers may be used. Blends of fibers may be used. The fibers may be bicomponent fibers. For example, the fibers may have a core of one polymer, and a sheath of a different polymer. The denier of the fibers comprising the wound-contacting pad is not limited, but typically ranges from about 3 to 10 denier.

The basis weight of the wound-contacting pad is not limited, but typically ranges from 0.003 g/cm² to 0.015 g/cm². The size of the wound-contacting pad may vary depending on the size of the bandage and/or the size of the wound to be protected or treated.

Typically, an adhesive is used to adhere the selectively placed layer to the backing material, to adhere the wound-contacting pad to the selectively placed layer, and to adhere the backing material to the skin of the consumer. The adhesives may be aqueous or solvent-based adhesives or they may be hot melt adhesives, as desired. Examples of suitable adhesives include, but are not limited to, those based on styrenic block copolymers and tackifying resins such as HL-1491 available from HB-Fuller Co. (St. Paul MN), H-2543 available from ATO-Findley (Wawatausa, WI), and Resyn 34-5534 available from National Starch & Chemical Company (Bridgewater, NJ). Ethylene copolymers, including ethylene vinyl acetate copolymers, are also useful as adhesives.

Suitable adhesives also include acrylic based, dextrin based, and urethane based adhesives as well as natural and synthetic elastomers. The adhesives may also include amorphous polyolefins including amorphous polypropylene, such as HL-1308 available from HB Fuller or Rextac RT 2373 available from Huntsman (Odesssa, TX). The adhesive may be based on Kraton® Brand synthetic elastomers, or natural rubber. These adhesives may also include tackifiers, anti-oxidants, processing oils, and the like as is known in the art.

The adhesive can be applied in any desired manner, e.g., by spraying, screen printing or slot die coating. The amount of adhesive typically applied is well known in the art, however generally, the adhesive coating weight may vary from about 20 grams per square meter ("gsm") to about 100 gsm.

In a second embodiment of an adhesive bandage according to the present invention, the selectively placed layer is a layer of wound-cushioning material which, when the bandage is used, provides improved cushioning for a wound or bruise. The selectively placed wound-cushioning material may be, for example, a polymeric foam such as a polyethylene foam, a polyurethane foam, or the like. The wound-cushioning layer may, if desired, be a woven or nonwoven fabric or a polymeric film having cushioning properties.

The present invention also provides a method for making an adhesive bandage. The method includes: providing a backing material; applying an adhesive to one major surface of the backing material; securing a selectively placed layer of material on the adhesive coated backing material; and securing a wound-contacting pad to the selectively placed layer. The wound-contacting pad can be secured to the selectively placed layer by the use of adhesive or heat-sealing. Optionally, the wound-contacting layer may be covered with a layer of wound release material, e.g., an open-mesh plastic netting.

Bandages in accordance with the invention may be square, rectangular, round, oval, or triangular in shape. The size of the bandage will depend on the shape of the bandage and the size of the wound meant to be covered by the bandage. Generally, a square bandage may range in size from 5 cm x 5 cm to 15 cm x 15 cm, preferably from 7.5 cm x 7.5 cm to 12.5 cm x 12.5 cm. The length of a rectangular bandage may range from 5 cm to 15 cm, preferably from 7,5 cm to 12.5 cm. The width of a rectangular bandage may range from 0.5 cm to 5 cm, preferably from 1 cm to 3 cm.

The thickness of the bandage of the invention will vary depending on the application, but generally may range from 0.25 mm to 5 mm, preferably 1 mm to 3 mm, more preferably 1 mm to 2 mm.

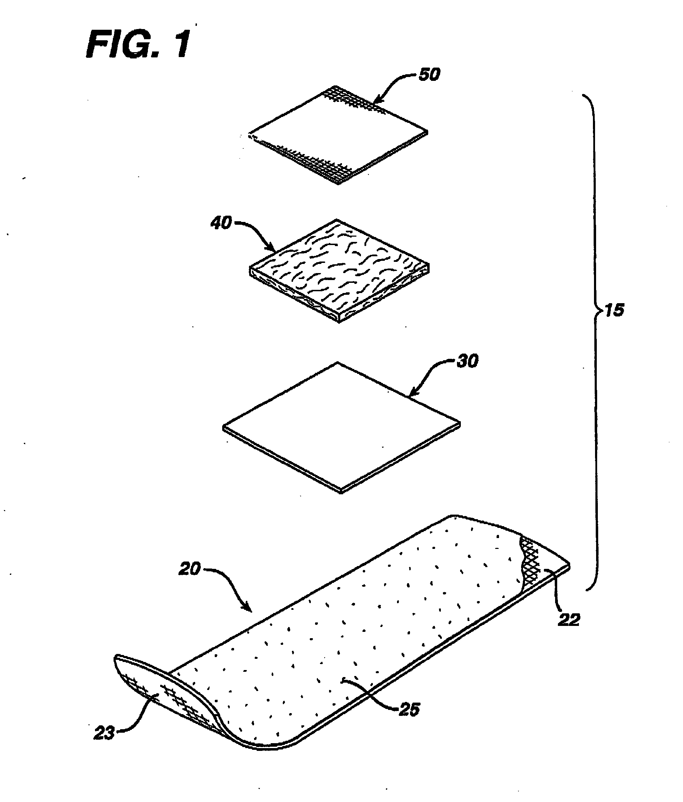

Referring now to the accompanying drawings, FIGS. 1-3 thereof illustrate one embodiment of an adhesive bandage in accordance with the present invention. Adhesive bandage 15 comprises a backing material 20, a selectively placed semi-permeable film 30, a wound-contacting pad 40 and a wound release layer 50. The wound release layer is optional, but is typically used in adhesive bandages of the invention to prevent undesirable adherence of the bandage to the underlying wound site.

In the embodiment of FIGS. 1-3, backing material 20 has a thickness of about 3 mils and has a first major surface 22 and a second major surface 23. Backing material 20 comprises a commercially available woven fabric having polyethylene terephthalate yarns in the warp direction and polybutylene terephthalate yarns in the fill direction. The warp yarns are single ply yarns, each yarn having a denier of 150. There are 84 warp yarns per 2.54 cm (84 warp yarns per inch). The fill yarns are two ply yarns. Each ply has a denier of 75, so that each two ply yarn has a denier of 150. Backing material 20 was treated by the supplier with a fluorocarbon water repellent to provide a spray rating of at least 80% as determined by ASTM C-1105 test procedure. Backing material 20 is permeable to water vapor and gases like oxygen. The moisture vapor transmission rate (MVTR) of this backing material is about 81,000 grams/square mater/24 hours. The thickness of backing 20 may range from 0.1 mm to about 5 mm, preferably 0.5 mm to 3 mm, even more preferably 0.5 mm to 2 mm.

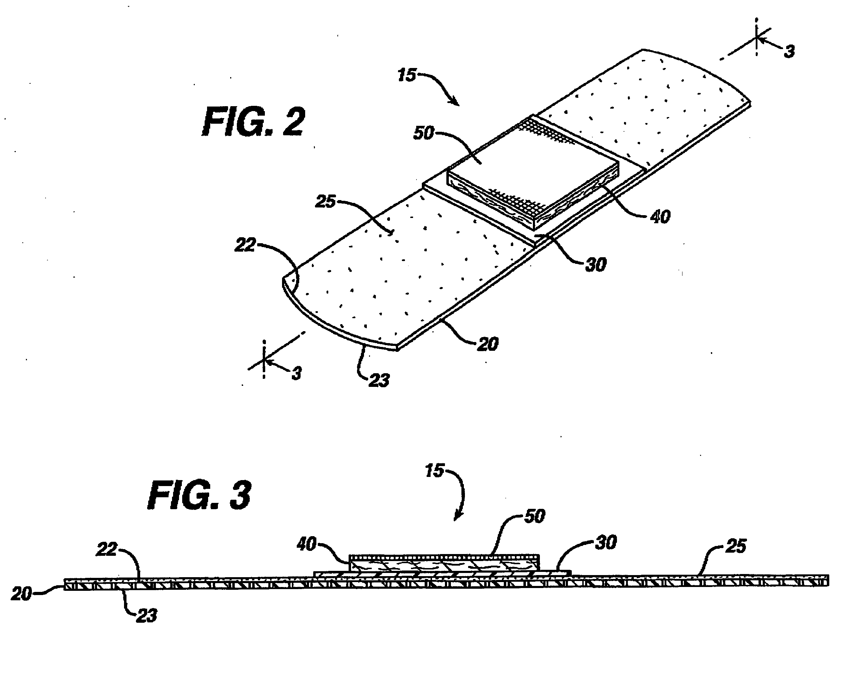

The first major surface 22 of the backing material carries a pressure sensitive hot melt adhesive of the type described generally in US 4,080,348. The adhesive is indicated by the stippling on first major surface 22 of backing material 20 in FIGS. 1 and 2 and by numeral 25 in 3. This adhesive is applied by slot die coating to the aforementioned woven backing at a level of about 60 grams of adhesive per square meter of fabric (60 gsm). Upon application, and evidently owing to the different chemical nature of the warp and fill yarns comprising the fabric, the hot melt adhesive adheres to some portions of the surface of the backing material but not to other portions. As a result, the backing material with the adhesive thereon is breathable, i.e., it allows for the passage of water vapor and other gases. Further information concerning this backing material can be found in U.S. Patent Application Serial No. 09/596,112..

A commercially available semi-permeable polyurethane film 30, approximately 1 mm thick, is placed on the adhesive coated first major surface 22 of backing material 20, thereby securing said semi-permeable film to said backing. This polyurethane film, which was obtained from J.P. Stevens under the designation Type 625, had an MVTR of about 25,000 grams/square meter/24 hours. As will be seen by reference to 2, semi-permeable film 30 has a width substantially equal to the width of backing material 20. However, as can be seen in FIG. 3, the length of semi-permeable film 30 is considerably less than the length of backing material 20 to which it is secured. Very typically, and as seen in FIGS. 2 and 3, semi-permeable film 30 is centered end-to-end of the backing material. Those skilled in the art will recognize, however, that this need not always be the case, and the semi-permeable film may, if desired, be offset toward one of the ends of the backing material.

Wound-contacting pad 40, which comprises a blend of 10% by weight rayon fibers and 90% polypropylene fibers is secured to upper surface of semi-permeable film 30. The basis weight of this wound-contacting pad 40 is 3.7 ounces/sq. yard, The securing of pad 40 to film 30 is preferably achieved by the use of an adhesive at an application rate sufficiently small so as to maintain breathability. An adhesive application rate of about 38 grams/square meter was used for securing wound-contacting pad 40 to film 30 in the bandage of FIGS. 1-3. Alternatively, where the wound-contacting pad comprises so-called "heat-sensitive" fibers, the wound-contacting pad may be heat-sealed, preferably in a discontinuous pattern, to the underlying selectively placed, semi-permeable film. Physical integrity of wound-contacting pad 40 is achieved by the frictional engagement of the fibers or blend of fibers of which it is constituted. Physical integrity of the wound-contacting pad may be enhanced, if necessary or desired, by the application thereto of a binder as is known in the art. It will be understood that if selectively placed, semi-permeable film 30 is secured to backing material 20 by heat sealing, the portions of the backing material adjacent semi-permeable film 30 will be coated with an adhesive in order to adhere the finished bandage to the skin of the user.

As can be seen by reference to FIGS. 2 and 3 of the drawings, the wound-contacting pad 40 of the specific adhesive bandage 15 under discussion has a length and width which are less than the length and width of semi-permeable film 30. This is a preferred, but not necessary, aspect of the invention.

Although not a necessary feature of the present invention, it is preferable to cover the upper surface of wound-contacting pad 40 with a wound release means 50. As is known in the art, an apertured plastic film or netting, e.g., one made from polyethylene or the like, may serve as wound release means 50. Apertured plastic films suitable for covering the wound-contacting pad are commercially available, e.g., from Applied Extrusion Technology, Middletown, Delaware 19709 USA.

Following are the dimensions of the structural components of a typical adhesive bandage of the type shown in FIGS. 1-3: length of backing material 20 = 7.5 cm; width of backing material 20 = 2.5 cm; length of semi-permeable film 30 = 2.5 cm; width of semi-permeable film 30 = 2.5 cm; length of wound-contacting pad 40 = 1.8 cm; width of wound-contacting pad 40 = 1.8 cm. The backing material of a bandage having the foregoing dimensions would therefore have an area of 18.75 square centimeters and the selectively placed semi-permeable film 30 would have an area of 6.25 square centimeters. Selectively placed layer 30 would therefore cover about 33% of the area of the backing layer 20. Wound-contacting pad 40 would have an area of about 3.24 square centimeters and would therefore cover about 51.7% of the area of the underlying selectively placed semi-permeable film 30. Wound-contacting pad 40 would cover about 17.3% of the area of backing material 20.

In addition to the above-described woven fabric, the backing material 20 of the adhesive bandage of FIGS. 1-3 may be a plastic film, e.g., a film of polyethylene, polypropylene or polyvinylchloride: Such films may be perforated to provide "breathability". Backing material 20 may alternatively comprise a nonwoven fabric or an open or closed cell foam. Semi-permeable films such as those made foam polyether amides or polyester amides may be used in place of the above-mentioned selectively placed, semi-permeable polyurethane film 30.

Quite advantageously, adhesive bandage 15 of FIGS. 1-3, with its woven backing material 20 and its smaller selectively placed, semi-permeable film 30, is considerably less expensive to manufacture than a corresponding adhesive bandage in which the backing material is made entirely from a semi-permeable film and has no selectively placed layer. This is because the combined cost of the woven backing material 20 and the selectively placed semi-permeable film 30, as illustrated in FIGS. 1-3, is considerably less than the cost of a semi-permeable film having a length corresponding to the length of woven backing material 30.

Bandage 15 of FIGS. 1-3 was tested and found to have an MVTR of about 16,000 grams/square meter/24 hrs.

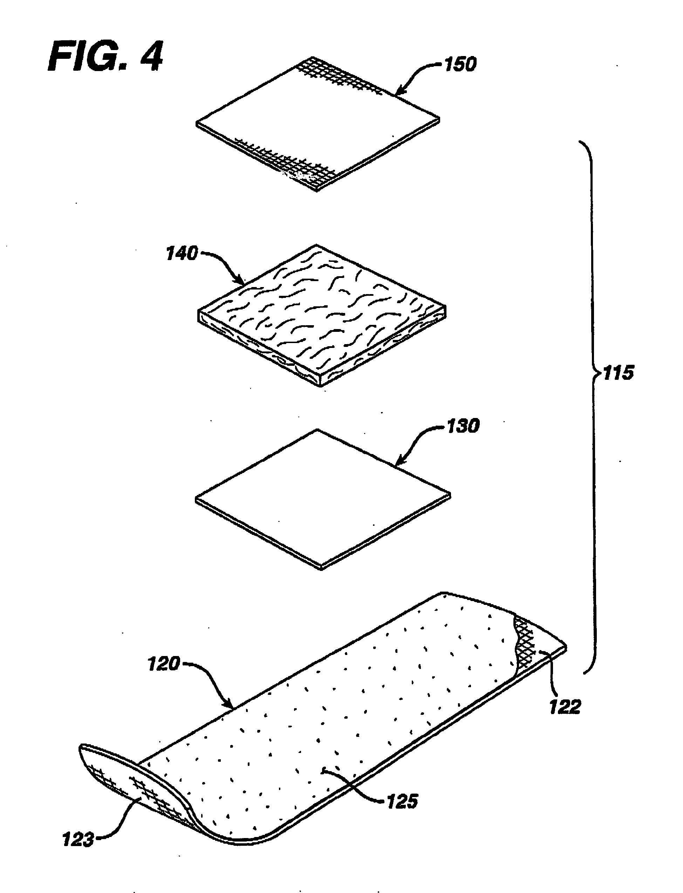

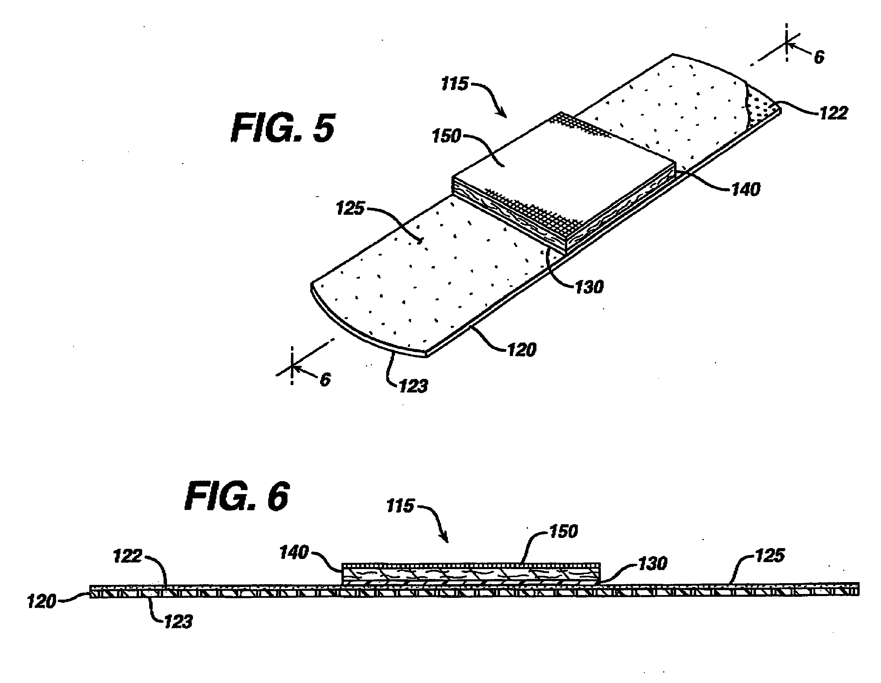

Another embodiment of an adhesive bandage in accordance with the present invention is shown in FIGS. 4-6 of the appended drawings. Bandage 115 comprises a backing material 120, an adhesive 125, a selectively placed semi-permeable film 130, a wound-contacting pad 140 and a wound release layer 150. In this embodiment, backing material 120, adhesive 125, semi-permeable film 130, wound-contacting pad 140, and optional wound release layer 150 are the same as structural elements 20, 25, 30, 40 and 50, respectively, of adhesive bandage 15 illustrated in FIGS. 1-3. Selectively placed semi-permeable film 130 has a length which is less than the length of backing 120 and a width which is substantially the same as the width of backing 120. As will be seen by reference to FIGS. 5 and 6, selectively placed layer 130 and wound-contacting pad 140 are co-extensive in length and width. If bandage 115 employs the same size wound-contacting pad as bandage 15, and assuming each of layers 30 and 130 is made of the same semi-permeable film, then bandage 115 would be less expensive to manufacture than bandage 15, since bandage 115 would employ a lesser amount of selectively placed semi-permeable film 130.

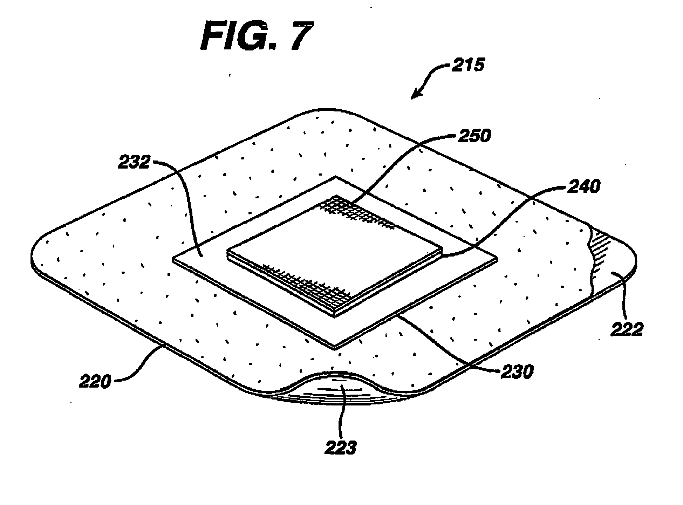

A further embodiment of an adhesive bandage in accordance with the present invention is illustrated in FIG. 7 Bandage 215 comprises a backing material 220 having a first major surface 222 and a second, opposed major surface 223. A selectively placed semi-permeable film 230 is secured, for example by heat-sealing or a suitable adhesive, to first major surface 222. First major surface 222 of the backing material is coated with a pressure sensitive adhesive (indicated by stippling in FIG. 7) in the regions thereof adjacent the perimeter of selectively placed, semi-permeable film 230. A wound-contacting pad 240 is secured, as by heat sealing or with a suitable adhesive, to the upper surface 232 of film 230. Wound-contacting pad 240 may be covered, if desired, by a porous wound release layer 250 of the kind described earlier herein. As can be seen in FIG. 7, wound-contacting pad 240 lies entirely within the perimeter of selectively placed, semi-permeable layer 230 and selectively placed layer 230 lies entirely within the perimeter of backing material 220. Although as illustrated in FIG. 7, bandage 215 is in the form of a square, it will be understood by those skilled in the art that the bandage may be provided in other geometric forms, for example, rectangular, circular or oval. Many variations of bandage 215 will be apparent to those skilled in the art. For example, backing 220, selectively placed layer 230 and wound-contacting pad 240 could be circular in configuration. It is also possible, of course, to provide bandage 215 with a backing 220 which has a square configuration and provide selectively placed layer 230 and wound-contacting pad 240 in circular configurations of diminishing size or in oval configurations of diminishing size. It will be understood that there is no particular requirement that backing 220, film 230 and wound-contacting pad 240 all have the same geometrical configuration. Variations other than those described above will be apparent to those skilled in the art.

Following are the dimensions of the structural components of a typical adhesive bandage of the type shown in FIG. 7: length and width of backing material 220 = 5.0 cm; length and width of selectively placed, semi-permeable film 230 = 2.5 cm; length and width of wound-contacting pad 240 = 1.8 cm The backing material 220 of a bandage having the foregoing dimensions would therefore have an area of 25 square centimeters and the selectively placed semi-permeable layer 230 would have an area of 6.25 square centimeters. Selectively placed layer 230 would therefore cover 25% of the area of the backing layer.

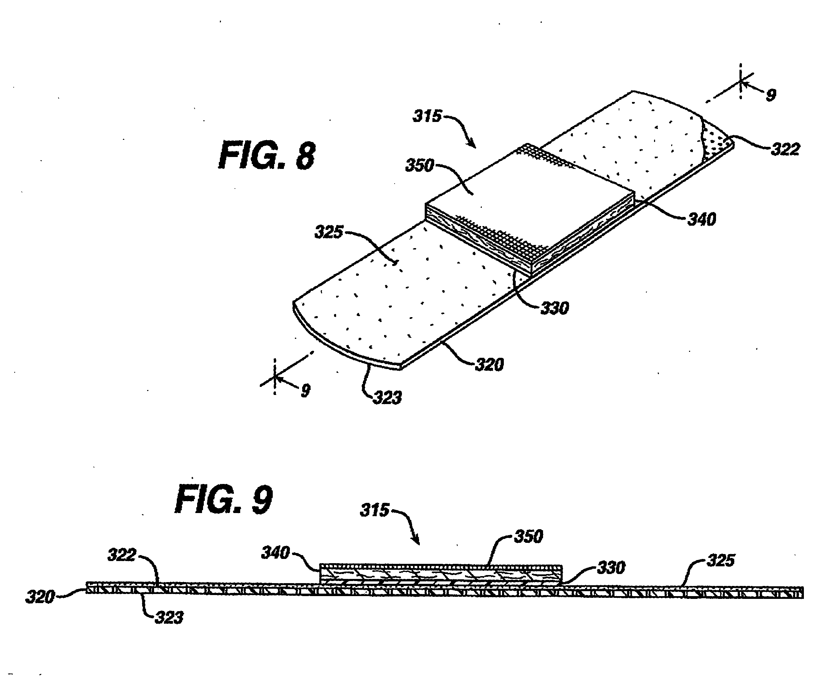

Referring now to FIGS. 8 and 9 of the drawings, there is illustrated an embodiment of an adhesive bandage according to the present invention in which the selectively placed layer comprises means for cushioning a wound to which the bandage is applied. Bandage 315 comprises a backing material 320 having a first major surface 322 and a second opposed major surface 323. First major surface 322 of the backing material has a pressure sensitive adhesive 325 applied thereto. A selectively placed layer of wound-cushioning material 330 is secured to first major surface 322 of backing 320 by a portion of adhesive 325. The remaining portions of adhesive 325 adjacent selectively placed layer 330 and the ends of the bandage are used to secure the bandage over the wound or bruise to be covered.

Wound-contacting pad 340 is placed over cushioning layer 330 and is secured thereto by any suitable means, e.g., by an adhesive. Wound release material 350, though not an essential element of the bandage, is typically placed over wound-contacting pad 340 and secured thereto, e.g., by heat-sealing or an adhesive. In the embodiment illustrated in FIGS. 8 and 9, selectively placed layer 330 has a length which is considerably smaller than the length of backing material 320 and a width which is substantially the same as the width of backing material 320. In the illustrated embodiment, wound-contacting pad 340 and wound release material 350 are co-extensive in length and width with selectively placed layer 330.

Wound-cushioning layer 330 may comprise any of the following materials; polymeric foams, woven fabrics, nonwoven fabrics, and polymeric films having cushioning properties.

Suitable polymeric foams include, but are not limited to, polyolefin foams, such as polyethylene and polypropylene foams; polyurethane foams, cellulosic foams, rubber foams, polyvinylchloride foams and the like. The foams may be open-cell foams or closed cell foams. They may be hydrophilic or hydrophobic, the latter usually being preferred. Useful foam thicknesses range from about 3 mm to about 6 mm but these dimensions are not critical. Foam thicknesses less than 3 mm or more than 6 mm may be useful as well. In any event, it will be understood that the thickness of the foam (as well as the thickness of the other cushioning materials mentioned above) will be of a thickness suitable for providing the desired cushioning function. The skilled art worker will be able to select suitable thicknesses.

Woven fabrics and nonwoven fabrics typically will have thicknesses in the same general range as those set forth above for polymeric foams.

Polymeric films are useful as cushioning materials where they have been treated, as by embossing, to have a thicknesses sufficient to provide the desired cushioning effect. Three dimensional polymeric films of the kind disclosed in U.S. 3,929,135 are useful in the practice of this embodiment of the invention.

As mentioned earlier herein, the selectively placed cushioning material 330 may comprise a bi-layer film which defines a pocket or, preferably, a plurality of small discontinuous pockets, in which air or other gas is entrapped

### Preparation of Bandages

Bandages according to the present invention can be made as follows: A strip of backing material having the desired dimensions is placed on a work surface and the selected adhesive is applied to one major surface thereof. The selectively place layer - either the semi-permeable film or the wound-cushioning material - is placed at the desired location on the adhesive surface of the backing material and pressed into place. The wound-contacting pad is then secured to the upper surface of the selectively placed layer by heat sealing or the use of an adhesive as desired. The wound release layer, if it is to be used, is secured to the upper surface of the wound-contacting pad. Release strips, e.g., siliconized paper, are placed over the exposed portions of adhesive as well as the selectively placed layer/wound-contacting pad/optimal wound release layer combination to protect the bandage prior to use. The bandage is then packaged in any convenient manner, for example by enclosing it between two layers of heat sealable paper and heat sealing the periphery of the two layers. The packaged bandage is then sterilized, if desired, by techniques well known in the art. The bandages can be made by hand or on commercially available bandage making equipment,

### In-Use Test

Bandages of the invention were tested as follows. Several subjects were asked to apply the adhesive bandages of FIGS. 1-3 around their fingers. Commercially available water impermeable bandages (in which a semi-permeable film is used as backing material) and regular bandages were also tested, The subjects placed their bandaged fingers in water containing food dye for 5 minutes. Bandages were then checked to see if water penetrated into the wound-contacting pad (as evidenced by the wound-contacting pad having the color of the food dye). If the wound-contacting pad did not have food dye in or on it, the bandage was considered to be waterproof. The results are shown in Table 1.

**Table 1**

| Bandage | Pass Percent |
|---|---|
| Example 1 - Adhesive bandage 15 (FIGS. 1-3) of the invention as described herein and comprising woven backing material 20, selectively placed semi-permeable polyurethane film 30, wound-contacting pad 40 and wound release layer 50. Backing material 20 comprises polyethylene terephthalate warp yarns and polybutylene terephthalate fill yarns. | 34 |
| Example 2 - Adhesive bandage comprising a non-perforated polyvinylchloride backing. Wound-contacting pad smaller in length and width than backing material. | 53 |
| Example 3 - Adhesive bandage sold commercially by 3M as CLEANS SEALS* Bandage. Backing material is a semi-permeable polyurethane film strip. Wound-contacting pad smaller in length and width than the backing material. | 56 |
| Example 4 - Adhesive bandage in which the backing material is a woven fabric having polyester warp yarns and nylon fill yarns. Same wound-contacting pad and wound release material as Example 1. This bandage has no selectively placed film. Configuration as shown in FIG. 5 of the drawings. | 3 |

The data above demonstrate that the adhesive bandage of the present invention (Example 1 in Table 1) is effective at preventing liquid water from penetrating through the bandage to the wound. Although the Example 1 bandages did not perform as well as the Example 2 bandages, it is believed that this may have been due to poor application of the bandages to the fingers, leaving gaps where water could penetrate through to the pad. On the other hand, the prior art adhesive bandage of Example 2 displayed considerable skin maceration owing to the fact that said bandage comprised a non-perforated, and therefore non-breathable, polyvinylchloride film as its backing material. The adhesive bandage of Example 1 in accordance with the invention displayed definitely less skin maceration, owing to the fact that the woven fabric used as the backing material 20 and the selectively placed semi-permeable film 30 are breathable.

## Claims

1. An adhesive bandage comprising:
A) a backing material (20/120/220/320),
B) an adhesive (25/125/325),
C) a selectively placed layer comprising a semi-permeable film (30/130/230/330), and
D) a wound-contacting pad (40/140/240/340);
each of said backing material, said semi-permeable film and said wound-contacting pad having a respective length and width;
said backing material having first (22, 122, 222, 322) and second (22, 123, 223, 323) major surfaces;
said adhesive being applied to said first major surface of said backing material;
said selectively placed layer being secured to said backing material by said adhesive;
at least the length of said selectively placed layer being less than the length of said backing to which it is secured;
said wound-contacting pad being secured to said selectively placed layer.

2. The adhesive bandage of claim 1 wherein the backing material is selected from the group consisting of polyolefin films, polyvinylchloride films, ethylene vinyl acetate films, polymeric foams, woven fabrics, nonwoven fabrics, laminates of polymeric films and woven fabrics, and laminates of polymeric films and nonwoven fabrics.

3. The adhesive bandage of claim 1 wherein the selectively placed layer covers a maximum of 86% of the backing material.

4. The adhesive bandage of claim 1 wherein the selectively placed layer covers a maximum of 70% of the backing material.

5. The adhesive bandage of claim 1 wherein the selectively placed layer covers a maximum of 53% of the backing material.

6. The adhesive bandage of claim 1 wherein the selectively placed semi-permeable film is selected from the group consisting of polyurethane films, polyolefin films, polyetheramide films and copolyester films.

7. The adhesive bandage of claim 6 wherein said semi-permeable film is a polyurethane film.

8. The adhesive bandage of Claim 1 wherein the width of said selectively placed layer is substantially the same as the width of said backing material.

9. The adhesive bandage of Claim 1 wherein the width of said selectively placed layer is less than the width of said backing material.

10. The adhesive bandage of claim 1 wherein the length and width of said selectively placed layer are less than the length and width of said backing material and said wound-contacting pad has a length which is less than the length of said selectively placed layer.

11. The adhesive bandage of claim 1 wherein the length and width of said selectively placed layer are less than the length and width of said backing layer and the length and width of said wound-contacting pad are less than the length and width of said selectively placed layer.

12. The adhesive bandage of to Claim 1 wherein said wound-contacting pad is substantially co-extensive with said selectively placed layer.

13. The adhesive bandage of claim 1 wherein the length of said wound-contacting pad is less than the length of said selectively placed layer.

14. The adhesive bandage of claim 1 wherein the width of said wound-contacting pad is less than the width of said selectively placed layer.

15. The adhesive bandage of claim 1 wherein the length and width of said wound-contacting pad is less than the length and width of said selectively placed layer.

16. An adhesive bandage according to claim 1 further comprising a wound release layer secured to said wound-contacting pad.

17. The adhesive bandage of claim 1 wherein said backing material is breathable.

18. The adhesive bandage of claim 1 wherein said backing material is selected from the group consisting of polyolefin films, polyvinylchloride films, ethylenevinyl acetate films and said films are perforated to make them breathable.

19. The adhesive bandage of claim 1 wherein said backing material and said adhesive are breathable.

20. The adhesive bandage of claim 1 wherein said adhesive is applied in a pattern to said backing material.

21. The adhesive bandage of claim 1 wherein said adhesive bandage is breathable.

22. The adhesive bandage of claim 21 further comprising a wound release layer secured to said wound-contacting pad.

23. The adhesive bandage of claim 9 wherein the length and width of said selectively placed layer are less than the length and width of said backing material, and said wound-contacting pad having a length and width less than the selectively placed layer.

24. The adhesive bandage of claim 23 wherein said backing material is breathable.

25. The adhesive bandage of claim 24 wherein said adhesive is breathable.

26. The adhesive bandage of claim 24 wherein said adhesive is applied in a pattern to said backing material.

27. The adhesive bandage of claim 1 wherein the selectively placed layer further comprises cushioning means (330) for cushioning a wound to which said bandage is applied.

28. The adhesive bandage of claim 27 wherein said wound-cushioning means (330) is selected from the group consisting of polymeric foams, woven fabrics, nonwoven fabrics, and polymeric films having cushioning properties.

29. The adhesive bandage of claim 28 wherein said polymeric foam is selected from the group consisting of polyolefin foams, polyurethane foams, rubber foams and polyvinylchloride foams.

30. The adhesive bandage of claim 27 wherein said wound-cushioning means comprises a polymeric film which has been treated to have a thickness sufficient to provide a wound-cushioning effect.

31. The adhesive bandage of claim 27 wherein said wound-cushioning material comprises a bi-layer film defining at least one pocket in which a gas is entrapped.

32. The adhesive bandage of claim 31 wherein said bi-layer film defines a plurality of small, discontinuous pockets in which a gas is entrapped.

33. The adhesive bandage of claim 27 wherein said wound-cushioning means and said wound-contacting pad are co-extensive in length and width.

34. The adhesive bandage of claim 27 further comprising a wound release layer secured to said wound-contacting pad.

35. The adhesive bandage of claim 27 wherein said backing material is breathable.

## Patentansprüche

1. Haftender Verband, der aufweist:
(A) ein Kaschiermaterial (20/120/220/320);
(B) ein Haftmittel (25/125/325);
(C) ein ausgewählt aufgebrachte Schicht, die einen halbdurchlässigen Film (30/130/230/330) aufweist; und
(D) ein die Wunde berührendes Kissen (40/140/240/340),
wobei sowohl das Kaschiermaterial, der halbdurchlässige Film als auch das die Wunde berührende Kissen eine jeweilige Länge und Breite haben;
wobei das Kaschiermaterial eine erste (22, 122, 222, 322) und eine zweite (23, 123, 223, 323) Hauptfläche hat;
wobei das Haftmittel auf die erste Hauptfläche des Kaschiermaterials aufgebracht ist;
wobei die ausgewählt aufgebrachte Schicht durch das Klebmittel an dem Kaschiermaterial gesichert ist;
wobei die Länge der ausgewählt aufgebrachten Schicht kleiner ist als die Länge der Kaschierung, an der sie gesichert ist;
wobei das die Wunde berührende Kissen an der ausgewählt aufgebrachten Schicht gesichert ist.

2. Haftender Verband nach Anspruch 1, bei dem das Kaschiermaterial aus der Gruppe bestehend aus Polyolefinfilmen, Polyvinylchloridfilmen, Ethylenvinylacetatfilmen, polymeren Schäumen, Geweben, Vliesen, Laminaten aus polymeren Filmen und Geweben und Laminaten aus polymeren Filmen und Vliesen ausgewählt ist.

3. Haftender Verband nach Anspruch 1, bei dem die ausgewählt aufgebrachte Schicht ein Maximum von 86 % des Kaschiermaterials abdeckt.

4. Haftender Verband nach Anspruch 1, bei dem die ausgewählt aufgebrachte Schicht ein Maximum von 70 % des Kaschiermaterials abdeckt.

5. Haftender Verband nach Anspruch 1, bei dem die ausgewählt aufgebrachte Schicht ein Maximum von 53 % des Kaschiermaterials abdeckt.

6. Haftender Verband nach Anspruch 1, bei dem der ausgewählt aufgebrachte halbdurchlässige Film aus der Gruppe bestehend aus Polyurethanfilmen, Polyolefinfilmen, Polyetheramidfilmen und Copolyesterfilmen ausgewählt ist.

7. Haftender Verband nach Anspruch 6, bei dem der halbdurchlässige Film ein Polyurethanfilm ist.

8. Haftender Verband nach Anspruch 1, bei dem die Breite der ausgewählt aufgebrachten Schicht im Wesentlichen dieselbe ist wie die Breite des Kaschiermaterials.

9. Haftender Verband nach Anspruch 1, bei dem die Breite der ausgewählt aufgebrachten Schicht kleiner ist als die Breite des Kaschiermaterials.

10. Haftender Verband nach Anspruch 1, bei dem die Länge und die Breite der ausgewählt aufgebrachten Schicht kleiner als die Länge und die Breite des Kaschiermaterials sind und das die Wunde berührende Kissen eine Länge hat, die kleiner ist als die Länge der ausgewählt aufgebrachten Schicht.

11. Haftender Verband nach Anspruch 1, bei dem die Länge und die Breite der ausgewählt aufgebrachten Schicht kleiner als die Länge und die Breite der Kaschierschicht sind und die Länge und die die Breite des die Wunde berührenden Kissens kleiner als die Länge und die Breite der ausgewählt aufgebrachten Schicht sind.

12. Haftender Verband nach Anspruch 1, bei dem das die Wunde berührenden Kissen der ausgewählt aufgebrachten Schicht in ihren Abmessungen im Wesentlichen entspricht.

13. Haftender Verband nach Anspruch 1, bei dem die Länge des die Wunde berührenden Kissens kleiner ist als die Länge der ausgewählt aufgebrachten Schicht.

14. Haftender Verband nach Anspruch 1, bei dem die Breite des die Wunde berührenden Kissens kleiner ist als die Breite der ausgewählt aufgebrachten Schicht.

15. Haftender Verband nach Anspruch 1, bei dem die Länge und die Breite des die Wunde berührenden Kissens kleiner sind als die Länge und die Breite der ausgewählt aufgebrachten Schicht.

16. Haftender Verband nach Anspruch 1, weiter mit einer Wundentrennschicht, die an dem die Wunde berührenden Kissen gesichert ist.

17. Haftender Verband nach Anspruch 1, bei dem das Kaschiermaterial atmungsaktiv ist.

18. Haftender Verband nach Anspruch 1, bei dem das Kaschiermaterial aus der Gruppe bestehend aus Polyolefinfilmen, Polyvinylchloridfilmen, Ethylenvinylacetatfilmen ausgewählt ist und die Filme perforiert sind, um sie atmungsaktiv zu machen.

19. Haftender Verband nach Anspruch 1, bei dem das Kaschiermaterial und das Haftmittel atmungsaktiv sind.

20. Haftender Verband nach Anspruch 1, bei dem das Haftmittel in einem Muster auf das Kaschiermaterial aufgebracht ist.

21. Haftender Verband nach Anspruch 1, wobei der haftende Verband atmungsaktiv ist.

22. Haftender Verband nach Anspruch 21, weiter mit einer Wundentrennschicht, die an dem die Wunde berührenden Kissen gesichert ist.

23. Haftender Verband nach Anspruch 9, bei dem die Länge und die Breite der ausgewählt aufgebrachten Schicht kleiner sind als die Länge und die Breite des Kaschiermaterials und das die Wunde berührende Kissen eine Länge und eine Breite kleiner als die ausgewählt aufgebrachte Schicht hat.

24. Haftender Verband nach Anspruch 23, bei dem das Kaschiermittel atmungsaktiv ist.

25. Haftender Verband nach Anspruch 24, bei dem das Haftmittel atmungsaktiv ist.

26. Haftender Verband nach Anspruch 24, bei dem das Haftmittel in einem Muster auf das Kaschiermaterial aufgebracht ist.

27. Haftender Verband nach Anspruch 1, bei dem die ausgewählt aufgebrachte Schicht weiter eine Polstereinrichtung (330) zum Abpolstern einer Wunde, auf die der Verband gelegt ist, aufweist.

28. Haftender Verband nach Anspruch 27, bei dem die die Wunde polsternde Einrichtung (330) aus der Gruppe bestehend aus polymeren Schäumen, Geweben, Vliesen und polymeren Filmen mit Polstereigenschaften ausgewählt ist.

29. Haftender Verband nach Anspruch 28, bei dem der polymere Schaum aus der Gruppe bestehend aus Polyolefinschäumen, Polyurethanschäumen, Gummischäumen und Polyvinylchloridschäumen ausgewählt ist.

30. Haftender Verband nach Anspruch 27, bei dem die die Wunde polsternde Einrichtung einen polymeren Film aufweist, der behandelt worden ist, so dass er eine Dicke hat, die ausreichend ist, um für eine die Wunde polsternde Wirkung zu sorgen.

31. Haftender Verband nach Anspruch 27, bei dem das die Wunde polsternde Material einen zweischichtigen Film aufweist, welcher wenigstens eine Tasche definiert, in der ein Gas eingefangen ist.

32. Haftender Verband nach Anspruch 31, bei dem der zweischichtige Film eine Vielzahl kleiner, nicht miteinander verbundener Taschen definiert, in denen ein Gas eingefangen ist.

33. Haftender Verband nach Anspruch 27, bei dem sich die die Wunde polsternde Einrichtung und das die Wunde berührende Kissen in Länge und Breite entsprechen.

34. Haftender Verband nach Anspruch 27, weiter mit einer Wundentrennschicht, die an dem die Wunde berührenden Kissen gesichert ist.

35. Haftender Verband nach Anspruch 27, bei dem das Kaschiermaterial atmungsaktiv ist.

## Revendications

1. Bandage adhésif comprenant :
A) un matériau d'envers (20/120/220/320),
B) un adhésif (25/125/325),
C) une couche placée sélectivement comprenant un film semi-perméable (30/130/230/330), et
D) une compresse pour contact avec une plaie (40/140/240/340) ;
chacun parmi ledit matériau d'envers, ledit film semi-perméable et ladite compresse pour contact avec une plaie ayant une longueur et une largeur respectives ;
ledit matériau d'envers ayant des première (22, 122, 222, 322) et deuxième (23, 123, 223, 323) surfaces majeures ;
ledit adhésif étant appliqué à ladite première surface majeure dudit matériau d'envers ;
ladite couche placée sélectivement étant fixée audit matériau d'envers par ledit adhésif ;
au moins la longueur de ladite couche placée sélectivement étant inférieure à la longueur dudit envers auquel elle est fixée ;
ladite compresse pour contact avec une plaie étant fixée à ladite couche placée sélectivement.

2. Bandage adhésif selon la revendication 1, dans lequel le matériau d'envers est choisi dans le groupe constitué par les films de polyoléfine, les films de poly(chlorure de vinyle), les films d'éthylène/acétate de vinyle, les mousses polymères, les étoffes tissées, les étoffes non-tissées, les stratifiés de films polymères et d'étoffes tissées, et les stratifiés de films polymères et d'étoffes non-tissées.

3. Bandage adhésif selon la revendication 1, dans lequel la couche placée sélectivement couvre un maximum de 86 % du matériau d'envers.

4. Bandage adhésif selon la revendication 1, dans lequel la couche placée sélectivement couvre un maximum de 70 % du matériau d'envers.

5. Bandage adhésif selon la revendication 1, dans lequel la couche placée sélectivement couvre un maximum de 53 % du matériau d'envers.

6. Bandage adhésif selon la revendication 1, dans lequel le film semi-perméable placé sélectivement est choisi dans le groupe constitué par les films de polyuréthane, les films de polyoléfine, les films de polyéther-amide et les films de copolyester.

7. Bandage adhésif selon la revendication 6, dans lequel ledit film semi-perméable est un film de polyuréthane.

8. Bandage adhésif selon la revendication 1, dans lequel la largeur de ladite couche placée sélectivement est pratiquement identique à la largeur dudit matériau d'envers.

9. Bandage adhésif selon la revendication 1, dans lequel la largeur de ladite couche placée sélectivement est inférieure à la largeur dudit matériau d'envers.

10. Bandage adhésif selon la revendication 1, dans lequel la longueur et la largeur de ladite couche placée sélectivement sont inférieures à la longueur et la largeur dudit matériau d'envers et ladite compresse pour contact avec une plaie a une longueur qui est inférieure à la longueur de ladite couche placée sélectivement.

11. Bandage adhésif selon la revendication 1, dans lequel la longueur et la largeur de ladite couche placée sélectivement sont inférieures à la longueur et la largeur de ladite couche d'envers et la longueur et la largeur de ladite compresse pour contact avec une plaie sont inférieures à la longueur et la largeur de ladite couche placée sélectivement.

12. Bandage adhésif selon la revendication 1, dans lequel ladite compresse pour contact avec une plaie est pratiquement coextensive avec ladite couche placée sélectivement.

13. Bandage adhésif selon la revendication 1, dans lequel la longueur de ladite compresse pour contact avec une plaie est inférieure à la longueur de ladite couche placée sélectivement.

14. Bandage adhésif selon la revendication 1, dans lequel la largeur de ladite compresse pour contact avec une plaie est inférieure à la largeur de ladite couche placée sélectivement.

15. Bandage adhésif selon la revendication 1, dans lequel la longueur et la largeur de ladite compresse pour contact avec une plaie sont inférieures à la longueur et la largeur de ladite couche placée sélectivement.

16. Bandage adhésif selon la revendication 1, comprenant en outre une couche n'adhérant pas à la plaie fixée à ladite compresse pour contact avec une plaie.

17. Bandage adhésif selon la revendication 1, dans lequel ledit matériau d'envers est perméable à l'air.

18. Bandage adhésif selon la revendication 1, dans lequel ledit matériau d'envers est choisi dans le groupe constitué par les films de polyoléfine, les films de poly(chlorure de vinyle), les films d'éthylène/acétate de vinyle et lesdits films sont perforés pour être rendus perméables à l'air.

19. Bandage adhésif selon la revendication 1, dans lequel ledit matériau d'envers et ledit adhésif sont perméables à l'air.

20. Bandage adhésif selon la revendication 1, dans lequel ledit adhésif est appliqué selon un motif audit matériau d'envers.

21. Bandage adhésif selon la revendication 1, lequel bandage adhésif est perméable à l'air.

22. Bandage adhésif selon la revendication 21, comprenant en outre une couche n'adhérant pas à la plaie fixée à ladite compresse pour contact avec une plaie.

23. Bandage adhésif selon la revendication 9, dans lequel la longueur et la largeur de ladite couche placée sélectivement sont inférieures à la longueur et la largeur dudit matériau d'envers, et ladite compresse pour contact avec une plaie a une longueur et une largeur inférieures à celles de la couche placée sélectivement.

24. Bandage adhésif selon la revendication 23, dans lequel ledit matériau d'envers est perméable à l'air.

25. Bandage adhésif selon la revendication 24, dans lequel ledit adhésif est perméable à l'air.

26. Bandage adhésif selon la revendication 24, dans lequel ledit adhésif est appliqué selon un motif audit matériau d'envers.

27. Bandage adhésif selon la revendication 1, dans lequel la couche placée sélectivement comprend en outre des moyens amortisseurs (330) pour matelasser une plaie à laquelle ledit bandage est appliqué.

28. Bandage adhésif selon la revendication 27, dans lequel lesdits moyens amortisseurs pour plaie (330) sont choisis dans le groupe constitué par les mousses polymères, les étoffes tissées, let étoffes non-tissées, et les films polymères ayant des propriétés amortisseuses.

29. Bandage adhésif selon la revendication 28, dans lequel ladite mousse polymère est choisie dans le groupe constitué par les mousses de polyoléfine, les mousses de polyuréthane, les mousses de caoutchouc et les mousses de poly(chlorure de vinyle).

30. Bandage adhésif selon la revendication 27, dans lequel lesdits moyens amortisseurs pour plaie comprennent un film polymère qui a été traité de façon à avoir une épaisseur suffisante pour conférer un effet d'amortissement pour la plaie.

31. Bandage adhésif selon la revendication 27, dans lequel ledit matériau amortisseur pour plaie comprend un film bicouche définissant au moins une poche dans laquelle un gaz est piégé.

32. Bandage adhésif selon la revendication 31, dans lequel ledit film bicouche définit une pluralité de petites poches discontinues dans lesquelles un gaz est piégé.

33. Bandage adhésif selon la revendication 27, dans lequel lesdits moyens amortisseurs pour plaie et ladite compresse pour contact avec une plaie sont coextensifs dans la longueur et dans la largeur.

34. Bandage adhésif selon la revendication 27, comprenant en outre une couche n'adhérant pas à la plaie fixée à ladite compresse pour contact avec une plaie.

35. Bandage adhésif selon la revendication 27, dans lequel ledit matériau d'envers est perméable à l'air.
